# EUROPEAN PATENT SPECIFICATION

(11) **EP 2 939 707 B1**
(45) Date of publication and mention of the grant of the patent: **06.06.2018**
(21) Application number: 15165014.0
(22) Date of filing: 24.04.2015
(51) Int. Cl.: A61N 1/39

(54) **MEDICAL APPARATUS**
MEDIZINISCHE VORRICHTUNG
APPAREIL MÉDICAL

(30) Priority: 28.04.2014 JP 2014092576
(43) Date of publication of application: 04.11.2015
(73) Proprietor: Nihon Kohden Corporation, Shinjuku-ku Tokyo 161-8560 (JP)
(72) Inventor: Wakabayashi, Tsutomu, Tokyo, 161-8560 (JP); Tsumura, Ikuhiro, Tokyo, 161-8560 (JP); Kubota, Takayuki, Tokyo, 161-8560 (JP)
(74) Representative: Hoffmann Eitle

(56) References cited:
- EP-A2- 0 757 912
- JP-A- 2001 154 928
- US-A1- 2004 133 244

## Description

### TECHICAL FIELD

The present invention relates to a medical apparatus and, more particularly, to a medical apparatus having an abnormality monitoring mechanism.

### BACKGROUND ART

In recent years, an AED (Automatic External Defibrillator), which analyzes a rescuee during occurrence of ventricular fibrillation and restores the function of the heart by applying an electric shock as necessary, has been widely used. An AED applies a strong electric shock to the heart that has stopped due to ventricular fibrillation to remove the spasm of the heart muscle. Since the operation of an AED is fatal to a rescuee, a failure that occurs in an AED needs to be recognized immediately.

A general AED is provided with a status indicator on the surface of the cabinet. Upon detecting an abnormality (failure) internally, the AED indicates an abnormal state on the status indicator.

For example, PTL 1 discloses the related art having an advanced failure detection technique. The defibrillator disclosed in PTL 1 incorporates a self-test system. This automatic self-test system automatically carries out a function test and calibration verification inspection in response to a test signal to be generated periodically. Then, the self-test system outputs the test result visually and audibly. Accordingly, the state of the defibrillation can be determined and reported automatically without user intervention.

US 2004/133244 A1, which discloses the most relevant prior art, relates to an AED including defibrillation circuitry housed within an enclosure, a first processor programmed to periodically test the operability of the defibrillation circuitry and a second processor in communication with the first processor. The AED further includes a visual indicator, such as a red/green LED, positioned at the exterior of the enclosure that is operatively connected to the second processor. The second processor is programmed to control the visual indicator in response to the periodic test results provided to it by the first processor.

EP 0 757 912 A2 relates to an automated external defibrillator (AED) which automatically performs self-tests on a daily and weekly basis. Tested functions include the presence and interconnection of defibrillator electrodes, battery charge state and the operability of the high voltage circuit. Visual and audible indicators are actuated to alert an operator if faults are identified.

JP 2001-154928 A relates to controller abnormality detection method in master/slave type control system. The controller periodically issues an existence confirmation command to a system bus as a bus command. An existence monitoring timer of the slave unit for monitoring the operation state of the controller is preset, an existence monitoring timer value is updated until the existence confirmation command is extracted next, and in the case if judging that time is up, it is judged that the controller is abnormal.

### CITATION LIST

### PATENT LITERATURE

PTL 1: JP-A-9-500798

### SUMMARY OF INVENTION

### TECHNICAL PROBLEM

As described above, an AED has a self-check function and, when detecting an abnormality, indicates it on the status indicator. An indicator control unit (processing unit for changing the indication of the status indicator) performs the operation monitoring another control unit and, when detecting an operation abnormality, changes the indication of the status indicator. However, when the indicator control unit fails, the indicator control unit may not control the status indicator appropriately. Accordingly, the failure cannot be reported to the user. That is, the abnormality monitoring function of a conventional medical apparatus is insufficient and an abnormal state may not be reported to the user appropriately.

The above problem is not limited to an AED and common to various types of medical apparatuses having a status indicator (display unit).

The invention addresses the above problems with a main object of providing a medical apparatus that appropriately indicates an abnormal state to the user.

### SOLUTION TO PROBLEM

The above object is accomplished by the medical apparatus defined in claim 1. The dependent claims describe advantageous embodiments.

A medical apparatus according to an aspect of the invention includes an output unit outputting whether the medical apparatus is in an abnormal state, a first control unit controlling the medical apparatus, and a monitoring unit detecting an operation abnormality in the first control unit, in which the first control unit performs operation monitoring of the monitoring unit and, when detecting an operation
abnormality, causes the output unit to output an abnormal state and the monitoring unit performs operation monitoring of the first control unit and, when detecting an operation abnormality, causes the output unit to output an abnormal state.

In the medical apparatus according to the present embodiment, the first control unit and the monitoring unit monitor the operation each other and, when detecting an operation abnormality, cause the output unit to output the abnormality. Since the first control unit and the monitoring unit monitor the operation each other, an operation abnormality can be detected more accurately as compared with a configuration in which one-way operation monitoring is performed. Since an operation abnormality can be detected accurately, an abnormal state can be reported immediately to the user of the medical apparatus.

According to the invention, there is provided a medical apparatus capable of appropriately reporting an abnormal state of an apparatus to the user.

### BRIEF DESCRIPTION OF DRAWINGS

[Fig. 1] Fig. 1 is a block diagram illustrating the structure of an AED 1 (medical apparatus) according to embodiment 1.
[Fig. 2] Fig. 2 is a specific example of email transmitted by a communication unit 5 according to embodiment 1.
[Fig. 3] Fig. 3 is a block diagram illustrating the structure of the AED (medical apparatus) 1 according to embodiment 1.
[Fig. 4] Fig. 4 is a timing chart illustrating the monitoring of an operation abnormality performed by a monitoring unit 15 and a power supply control unit 8 according to embodiment 1 each other.
[Fig. 5] Fig. 5 is a timing chart illustrating the monitoring of an operation abnormality performed by an operation control unit 7 and the power supply control unit 8 according to embodiment 1 each other.

### REFERENCE SIGNS LIST

- 1: AED(Automated External Defibrillator)
- 2: power supply unit
- 3: DC/DC power supply unit
- 4: storage unit
- 5: communication unit
- 6: manipulating unit
- 7: operation control unit
- 8: power supply control unit
- 9: indicator
- 10: voice unit
- 11: electrocardiogram signal amplifying unit
- 12: high voltage generating unit
- 13: high voltage capacitor
- 14: pad connector
- 15: monitoring unit
- 16: internal battery
- 17: internal battery

### DESCRIPTION OF EMBODIMENTS

### <Embodiment 1>

A medical apparatus according to embodiment 1 will be described with reference to the drawings. As a medical apparatus according to the invention, a medical apparatus including batteries (first battery and second battery) capable of continuously supplying power and an attachable and detachable battery (removal battery) is mainly assumed. In the following description, this medical apparatus is assumed to be an AED (Automated External Defibrillator).

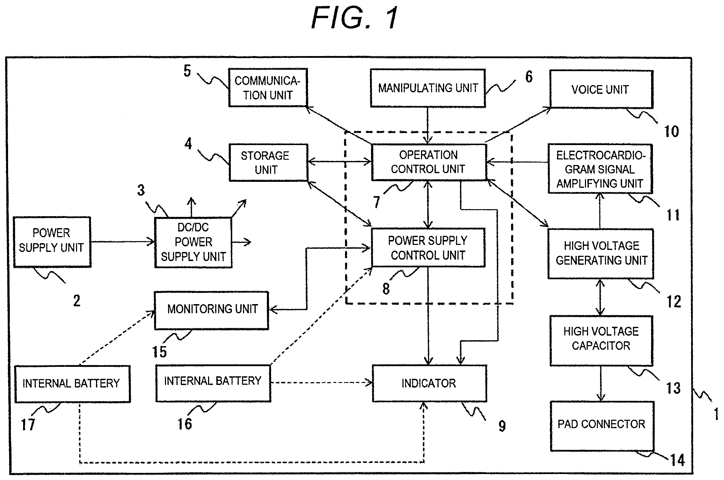

Fig. 1 is a block diagram illustrating the structure of an AED 1 (medical apparatus) according to the embodiment. The AED 1 includes a plurality of batteries: an internal battery 16 (first battery), an internal battery 17 (second battery), and a power supply unit 2 (removable battery). More specifically, the AED 1 includes the power supply unit 2 (removable battery), a DC/DC power supply unit 3, a storage unit 4, a communication unit 5, a manipulating unit 6, an operation control unit 7 (second control unit), a power supply control unit 8 (first control unit), an indicator 9 (which is a display unit and also an aspect of the output unit), a voice unit 10, an electrocardiogram signal amplifying unit 11, a high voltage generating unit 12, a high voltage capacitor 13, a pad connector 14, a monitoring unit 15, the internal battery 16 (first battery), and the internal battery 17 (second battery) .

The power supply unit 2 (removable battery) is a battery attachable to or detachable from the AED 1 and, for example, a lithium primary battery. The power supply unit 2 is a battery that has a larger capacity and a larger energy density than the internal battery 16 (first battery) and the internal battery 17 (second battery), which will be described later. The power supply unit 2 is preferably operable for several years without being replaced. The power supply unit 2 supplies power to processing units in the AED 1 via the DC/DC power supply unit 3.

The DC/DC power supply unit 3 converts a power voltage supplied from the power supply unit 2 to voltages necessary for circuits in the AED 1 and supplies the converted voltages to the circuits. The DC/DC power supply unit 3 performs voltage conversion using a technique of a switching regulator or series regulator.

The storage unit 4 stores programs necessary for operation of the AED 1, voice data, adjustment values specific to the medical apparatus, electrocardiogram data during rescue, and so on. The storage unit 4 is a secondary storage device such as, for example, a hard disk drive.

The communication unit 5 is a communication interface for transmitting electrocardiogram data during rescue and so on stored in the storage unit 4 to any computers. The manipulating unit 6 is an operation interface including a power button and shock button provided on a surface of the cabinet of the AED 1. The rescuer applies an electric shock to the rescuee by manipulating the manipulating unit 6.

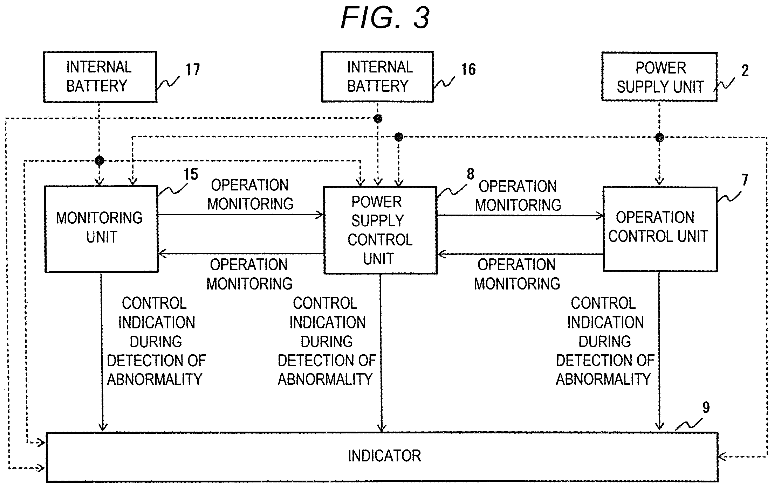

The operation control unit 7 (second control unit) controls the medical apparatus (that is, the AED 1). More specifically, the operation control unit 7 performs various types of operation control such as control of energy charging and discharging, sequence control, electrocardiogram analysis, and voice output control. The operation control unit 7 includes a CPU (Central Processing Unit), a gate array, an analog-to-digital converter, and so on. The operation control unit 7 also performs the operation monitoring of the power supply control unit 8 which will be described later and, when detecting an operation abnormality of the power supply control unit 8, performs control so that the indicator 9 indicates an abnormal state. Details on the operation monitoring will be described later with reference to Fig. 3 and other drawings.

The power supply control unit 8 (first control unit) is operable by receiving power from the internal battery 16 (which is not detachable from the AED 1 and capable of continuously supplying power). When power can be supplied from the power supply unit 2, the power supply control unit 8 may be operated by receiving power from the power supply unit 2 instead of the internal battery 16. This enables the power supply control unit 8 to be operated by receiving power from a stable power supply source (power supply unit 2) with larger capacity.

The power supply control unit 8 controls the medical apparatus (that is, the AED 1). More specifically, the power supply control unit 8 controls power supplied from the power supply unit 2, which is used as the main battery during a periodical inspection or the like. In addition, the power supply control unit 8 performs the operation monitoring of the operation control unit 7 and, when detecting an operation abnormality, switches the indication of the indicator 9. In addition, the power supply control unit 8 performs the operation monitoring of the monitoring unit 15 and, when detecting an operation abnormality, switches the indication of the indicator 9. The operation monitoring by the power supply control unit 8 will be described later with reference to Figs. 3 to 5.

Each of the operation control unit 7 (second control unit) and the power supply control unit 8 (first control unit) has a CPU (Central Processing Unit) and reads various types of programs from the storage unit 4 to execute them.

The indicator 9 (which is a display unit and also an aspect of the output unit) indicates the operating state of the AED 1. The indicator 9 reports, to the operator, the operating state of the AED 1 by, for example, the colors of lamps. When the power supply unit 2 with a large capacity can supply power, the indicator 9 operates by receiving power from the power supply unit 2 . When the power supply unit 2 cannot supply power (for example, the power supply unit 2 is removed from the AED 1), the indicator 9 operates by receiving power from the internal battery 16 or the internal battery 17.

The voice unit 10 outputs a voice (an instruction for the operator or a buzzer sound during detection of an abnormality) depending on control by the operation control unit 7. The electrocardiogram signal amplifying unit 11 filters and amplifies an electrocardiogram signal captured through a defibrillation pad (not illustrated) connected to the pad connector 14. The high voltage generating unit 12 charges or discharges energy used for defibrillation based on control by the operation control unit 7. The energy for defibrillation is charged in the high voltage capacitor 13. The pad connector 14 is an interface to the defibrillation pad (not illustrated) that makes contact with the body of the rescuee.

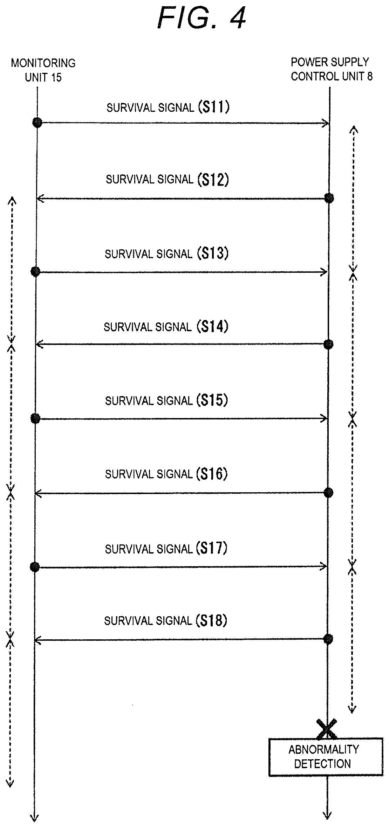

The monitoring unit 15 performs the operation monitoring of the power supply control unit 8 and, when detecting an operation abnormality, performs control so that the indicator 9 indicates an abnormality notification. The method for detecting an operation abnormality will be described later with reference to Fig. 4.

When the power supply unit 2 with a large capacity can supply power, the monitoring unit 15 operates by receiving power from the power supply unit 2. When the power supply unit 2 cannot supply power (for example, the power supply unit 2 is removed from the AED 1), the monitoring unit 15 operates by receiving power from the internal battery 17.

The internal battery 16 is a battery (not removed from the AED 1) in the AED 1 capable of continuously supplying power. The internal battery 16 supplies power to the power supply control unit 8 and the indicator 9. Similarly, the internal battery 17 is a battery (not removed from the AED 1) in the AED 1 capable of continuously supplying power. The internal battery 17 supplies power to the indicator 9 and the monitoring unit 15. That is, in the embodiment, the internal battery 16 and the internal battery 17 constitute a continuous power supply system.

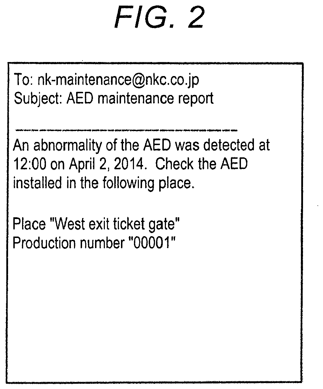

The communication unit 5, the voice unit 10, and the indicator 9 described above can be considered as output units that output an abnormal state within the AED 1. When the storage unit 4 is readable, the communication unit 5 may report occurrence of an abnormality by email (illustrated in Fig. 2, for example) to a predetermined address during detection of the abnormality. The destination address or the like only needs to be stored in the storage unit 4.

Alternatively, the voice unit 10 may report an abnormal state using a voice output during detection of an abnormality. As described above, the indicator 9 reports an abnormal state using indication of lamps or the like on the cabinet when detecting an abnormality. These output units may operate at the same time to report an abnormal state. For example, the voice unit 10 may perform a voice output indicating an abnormal state as soon as the indication of the indicator 9 is switched to an abnormal state.

Since the indicator 9 can be operated by receiving power from the internal battery 16 or the internal battery 17, even when an abnormal state is detected at a timing at which power is not supplied from the power supply unit 2, an appropriate report can be issued. In the following description, the indicator 9 is assumed to output an abnormal state.

Next, the relationship between the processing units concerning operation abnormality monitoring will be further described with reference to Fig. 3. The power supply unit 2 supplies power to the monitoring unit 15, the power supply control unit 8, and the operation control unit 7. When the power supply unit 2 cannot supply power, the internal battery 16 supplies power to the power supply control unit 8. When the power supply unit 2 cannot supply power, the internal battery 17 supplies power to the monitoring unit 15. Even when the power supply unit 2 does not operate, the indicator 9 receives power from the internal battery 16 or the internal battery 17. This makes the monitoring unit 15, the power supply control unit 8, and the indicator 9 operable at all times.

The monitoring unit 15 and the power supply control unit 8 monitor an operation abnormality each other and, when detecting an operation abnormality, switch the indication of the indicator 9. The power supply control unit 8 and the operation control unit 7 monitor an operation abnormality each other and, when detecting an operation abnormality, switch the indication of the indicator 9. Accordingly, when any of processing units is put in an abnormal state, the indicator 9 indicates the abnormal state. That is, in the AED 1, the processing units centering on the power supply control unit 8 monitor an operation abnormality each other and, when detecting an abnormality in any of them, the indicator 9 indicates the abnormality.

Next, the monitoring of an operation abnormality between the monitoring unit 15 and the power supply control unit 8 will be described in detail with reference to Fig. 4. The monitoring unit 15 periodically transmits a survival signal to the power supply control unit 8. The survival signal is a signal, having a small amount (number of bits) of information, that can be operated sufficiently in a power saving mode.

When receiving the survival signal periodically from the monitoring unit 15, the power supply control unit 8 determines that the monitoring unit 15 is normal. When not receiving the survival signal periodically from the monitoring unit 15 (that is, when not receiving the survival signal from the monitoring unit 15 again within a predetermined time after the previous reception of the survival signal), the power supply control unit 8 determines that the operation of the monitoring unit 15 is abnormal.

Similarly, when receiving the survival signal periodically from the power supply control unit 8, the monitoring unit 15 determines that the power supply control unit 8 is normal. When not receiving the survival signal periodically from the power supply control unit 8 (that is, when not receiving the survival signal from the power supply control unit 8 again within a predetermined time after the previous reception of the survival signal), the monitoring unit 15 determines that the operation of the power supply control unit 8 is abnormal. The intervals at which the monitoring unit 15 and the power supply control unit 8 transmit the survival signal only need to be, for example, one second.

Since the power supply control unit 8 has received survival signals S11, S13, S15, and S17 in the example in Fig. 4, the power supply control unit 8 determines that the monitoring unit 15 is normal in this period. Similarly, the monitoring unit 15 has received survival signal S12, S14, S16, and S18, the monitoring unit 15 determines that the power supply control unit 8 is normal in this period. The power supply control unit 8 has not received the survival signal within a predetermined time (for example, one second) after reception of the survival signal S17. Accordingly, the power supply control unit 8 determines that an operation abnormality has occurred in the monitoring unit 15. Upon detecting an operation abnormality, the power supply control unit 8 switches the indication of the indicator 9 to an abnormal state.

The monitoring unit 15 and the power supply control unit 8 may be operated by the internal battery 16 or the internal battery 17 with a small power supply capacity. Since the capacity of the internal battery 16 or the internal battery 17 is limited generally, high power consumption use is not preferable. On the other hand, operation monitoring between the monitoring unit 15 and the power supply control unit 8 is performed only by reception acknowledgement of a survival signal having a small amount (number of bits) of information. In other wards, the monitoring unit 15 and the power supply control unit 8 detect an operation abnormality without performing communication requiring many interactions such as so-called ACK (acknowledgement) or NACK (negative acknowledgement). Accordingly, the power consumption of the internal battery 16 can be suppressed and the life of the entire apparatus can be increased.

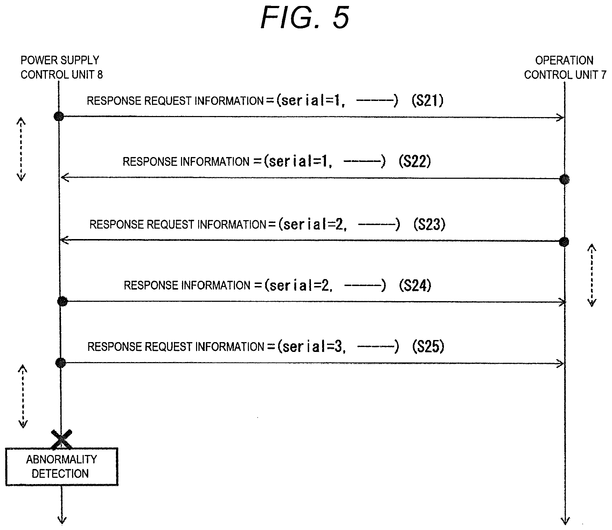

Next, operation monitoring between the operation control unit 7 and the power supply control unit 8 will be described with reference to Fig. 5. The power supply control unit 8 transmits response request information to the operation control unit 7. Response request information may include various types of information such as, for example, a serial number and requests of various computation results. The operation control unit 7 receives this response request information and transmits response information corresponding to the response request information to the power supply control unit 8. When the power supply control unit 8 does not receive response information within a predetermined time after transmitting the response request information or when the response request information does not correspond to the response information (for example, there is a mismatch in the serial number between the response request information and the response information or the calculation result is not the desired one), the power supply control unit 8 determines that an operation abnormality has occurred in the operation control unit 7.

The operation control unit 7 detects an operation abnormality in the power supply control unit 8 using a similar method (transmission and reception of response request information and response information). Upon detecting an abnormality, the operation control unit 7 switches the indication of the indicator 9.

In the example in Fig. 5, the power supply control unit 8 has not received response information from the operation control unit 7 within a predetermined time after transmitting the response request information S25. Accordingly, the power supply control unit 8 determines that an operation abnormality has occurred in the operation control unit 7. When determining that an operation abnormality has occurred, the power supply control unit 8 switches the indication of the indicator 9 to an abnormal state.

As described above, the operation control unit 7 is operated by receiving power from the power supply unit 2. The power supply control unit 8 is also operated by receiving power from the power supply unit 2 when the power supply unit 2 can supply power. That is, when abnormality detection is performed between these two processing units, it is expected that power is supplied from the power supply unit 2. By performing abnormality detection through transmission and reception of response request information and response information as described above in this situation, operation abnormality detection can be performed in more detail. That is, the operation control unit 7 and the power supply control unit 8 can detect an operation abnormality based on not only whether transmission and reception have been performed, but also whether appropriate calculation or the like can be performed.

Next, effects of the AED 1 (medical apparatus) according to the embodiment will be described. In the AED 1 according to the embodiment, the power supply control unit 8 (first control unit) and the monitoring unit 15 monitor the operation each other and, when detecting an operation abnormality, makes control so that the indicator 9 (aspect of the output unit performing abnormality output) performs an indication of an abnormality. Since the power supply control unit 8 and the monitoring unit 15 monitor the operation each other, an operation abnormality can be detected more accurately as compared with a configuration in which one-way operation monitoring is performed. Since an operation abnormality can be detected accurately, an abnormal state can be reported immediately to the user of the AED 1. This can reduce cases in which the AED 1 is left unrepaired even though the AED 1 has an abnormality internally.

Particularly, in the above configuration, the power supply control unit 8, the indicator 9, and the monitoring unit 15 are operable by receiving power from the internal battery 16 and 17 capable of continuously supplying power. Accordingly, even when the power supply unit 2 is removed, the operation of the power supply control unit 8 can be monitored and, when an operation abnormality occurs, the indicator 9 can indicate the abnormality.

The operation control unit 7 (second control unit) and the power supply control unit 8 (first control unit) also perform operation monitoring each other in the configuration. That is, the three processing units centering on the power supply control unit 8 perform monitoring each other (Fig. 3), so that an operation abnormality can be detected and indicated (output) more accurately.

The power supply control unit 8, the indicator 9, and the monitoring unit 15 are operable by the internal battery 16 (first battery) or the internal battery 17 (second battery) in the configuration. However, when the power supply unit 2 (removable battery) is available, the power supply control unit 8, the indicator 9, and the monitoring unit 15 are operated by receiving power from the power supply unit 2. When both the internal batteries 16 and 17 and the power supply unit 2 can be used, the power supply unit 2 with a larger capacity is used preferentially to prolong the battery life of the entire apparatus.

As described above, operation monitoring between the power supply control unit 8 and the monitoring unit 15 is performed preferably based on whether a survival signal with a small amount of information (number of bits) has been received (Fig. 4). By performing an operation check using only reception confirmation of the minimum amount of data, the power consumption of the internal battery 16 and 17 can be minimized.

The invention can be considered as follows. The AED 1 according to the embodiment has the monitoring unit 15 that is not present in the conventional configuration. The monitoring unit 15 is operated by a continuous power supply system and monitors the power supply control unit 8. That is, the AED 1 according to the embodiment has, in the continuous power supply system, a mechanism (monitoring unit 15) for monitoring an operation abnormality. Accordingly, even when the power supply unit 2 is removed, an operation abnormality can be detected. In addition, since the power supply control unit 8 monitors an operation abnormality in monitoring unit 15, abnormality detection can be performed more accurately. This can reduce cases in which the AED 1 is left unrepaired even though the AED 1 has an abnormality internally.

Although the invention devised by the inventor has been described specifically above based on the embodiment, the invention is not limited to the embodiment described above and it will be appreciated that various changes may be made without departing from the spirit of the invention.

For example, in the description of the configuration in Fig. 3, the monitoring unit 15 and the power supply control unit 8 can also receive power from the power supply unit 2, but each of these processing units may receive power from only one power supply source. In addition, the indicator 9 may indicate an abnormal state using characters instead of lamps.

In addition, in the description of the configuration, the power supply control unit 8 and the operation control unit 7 perform operation monitoring each other. However, even when at least one of the control units monitors the other, more sufficient effects can be expected than in the conventional configuration. Similarly, in the above description of the configuration, both the power supply control unit 8 and the monitoring unit 15 can use both the power supply unit 2 and the internal battery 16, but the invention is not limited to the configuration and sufficient effects can be expected even when at least one of the power supply control unit 8 and the monitoring unit 15 can use both a detachable power supply system and the continuous power supply system. In addition, the power supply control unit 8 and the monitoring unit 15 preferably use different internal batteries for stable operation, but the power supply control unit 8 and the monitoring unit 15 may theoretically use the same internal battery.

## Claims

1. A medical apparatus (1) comprising:
an output unit (9) for outputting whether the medical apparatus (1) is in an abnormal state;
a first control unit (8) for controlling the medical apparatus (1); and
a monitoring unit (15) for detecting an operation abnormality in the first control unit (8);
wherein the first control unit (8) is configured to perform operation monitoring of the monitoring unit (15) and, when detecting an operation abnormality, cause the output unit (9) to output an abnormal state,
the monitoring unit (15) is configured to perform operation monitoring of the first control unit (8) and, when detecting an operation abnormality, cause the output unit (9) to output an abnormal state,
the first control unit (8), the monitoring unit (15), and the output unit (9) are processing units operable by a power supply system capable of continuously supplying power,
the first control unit (8) is operable by a first battery (16) capable of continuously supplying power or an attachable and detachable removable battery (2),
the monitoring unit (15) is operable by a second battery (17) capable of continuously supplying power or the removable battery (2),
the medical apparatus (1) further comprises a second control unit (7) operable by the removable battery (2), the second control unit (7) controlling the medical apparatus (1), and
at least one of the first control unit (8) and the second control unit (7) is configured to perform operation monitoring of the other control unit and, when detecting an operation abnormality, cause the output unit (9) to output an abnormal state.

2. The medical apparatus according to claim 1, wherein the output unit (9) is operable by the first battery (16), the second battery (17), or the removable battery (2).

3. The medical apparatus (1) according to any one of claims 1 to 2,
wherein the first control unit (8) is configured for being operated by the removable battery (2) instead of the first battery (16) when the removable battery (2) is available.

4. The medical apparatus (1) according to any one of claims 1 to 3,
wherein at least one of the first control unit (8) and the monitoring unit (15) is configured to determine that an operation abnormality has occurred in the other unit when not receiving a periodical survival signal from the other unit.

5. The medical apparatus (1) according to claim 4,
wherein the first control unit (8) is configured to transmit response request information to the second control unit (7) and, when not receiving appropriate response information corresponding to the response request information from the second control unit (7) at a predetermined timing, determine that an operation abnormality has occurred in the second control unit (7).

## Patentansprüche

1. Medizinische Vorrichtung (1), umfassend:
eine Ausgabeeinheit (9) zur Ausgabe, ob die medizinische Vorrichtung (1) sich in einem anormalen Zustand befindet;
eine erste Steuereinheit (8) zum Steuern der medizinischen Vorrichtung (1); und
eine Überwachungseinheit (15) zum Erfassen einer Funktionsanomalität in der ersten Steuereinheit (8);
wobei die erste Steuereinheit (8) zur Ausführung einer Funktionsüberwachung der Überwachungseinheit (15) ausgelegt ist und wenn sie eine Funktionsanomalität erfasst, die Ausgabeeinheit (9) zur Ausgabe eines anormalen Zustands veranlasst,
die Überwachungseinheit (15) zur Ausführung einer Funktionsüberwachung der ersten Steuereinheit (8) ausgelegt ist und wenn sie eine Funktionsanomalität erfasst, die Ausgabeeinheit (9) zur Ausgabe eines anormalen Zustands veranlasst,
die erste Steuereinheit (8), die Überwachungseinheit (15) und die Ausgabeeinheit (9) Verarbeitungseinheiten sind, die durch ein Stromversorgungssystem funktionsfähig sind, das zur kontinuierlichen Stromversorgung in der Lage ist,
die erste Steuereinheit (8) durch eine erste Batterie (16), die zur kontinuierlichen Stromversorgung in der Lage ist, oder eine anbringbare und abnehmbare auswechselbare Batterie (2) funktionsfähig ist,
die Überwachungseinheit (15) durch eine zweite Batterie (17), die zur kontinuierlichen Stromversorgung in der Lage ist, oder die auswechselbare Batterie (2), funktionsfähig ist,
die medizinische Vorrichtung (1) weiter eine zweite Steuereinheit (7), die durch die auswechselbare Batterie (2) funktionsfähig ist, umfasst, wobei die zweite Steuereinheit (7) die medizinische Vorrichtung (1) steuert, und
mindestens eine von der ersten Steuereinheit (8) und der zweiten Steuereinheit (7) zur Ausführung einer Funktionsüberwachung der anderen Steuereinheit ausgelegt ist und wenn sie eine Funktionsanomalität erfasst, die Ausgabeeinheit (9) zur Ausgabe eines anormalen Zustands veranlasst.

2. Medizinische Vorrichtung nach Anspruch 1, wobei die Ausgabeeinheit (9) durch die erste Batterie (16), die zweite Batterie (17) oder die auswechselbare Batterie (2) funktionsfähig ist.

3. Medizinische Vorrichtung (1) nach einem der Ansprüche 1 bis 2,
wobei die erste Steuereinheit (8) ausgelegt ist, um durch die auswechselbare Batterie (2) anstelle der ersten Batterie (16) funktionsfähig zu sein, wenn die auswechselbare Batterie (2) verfügbar ist.

4. Medizinische Vorrichtung (1) nach einem der Ansprüche1 bis 3,
wobei mindestens eine von der ersten Steuereinheit (8) und der Überwachungseinheit (15) zum Bestimmen ausgelegt ist, dass eine Funktionsanomalität in der anderen Einheit aufgetreten ist, wenn kein periodisches Fortbestandssignal von der anderen Einheit empfangen wurde.

5. Medizinische Vorrichtung (1) nach Anspruch 4,
wobei die erste Steuereinheit (8) zur Übertragung einer Reaktionsanfrageinformation zur zweiten Steuereinheit (7) ausgelegt ist und wenn keine zutreffende Reaktionsinformation entsprechend der Reaktionsanfrageinformation aus der zweiten Steuereinheit (7) bei einer vorbestimmten Taktung empfangen wurde, Bestimmen, dass eine Funktionsanomalität in der zweiten Steuereinheit (7) aufgetreten ist.

## Revendications

1. Appareil médical (1) comprenant :
une unité de sortie (9) pour sortir si l'appareil médical (1) est dans un état anormal ;
une première unité de commande (8) pour commander l'appareil médical (1) ; et
une unité de contrôle (15) pour détecter une anomalie de fonctionnement dans la première unité de commande (8) ;
dans lequel la première unité de commande (8) est configurée pour effectuer un contrôle de fonctionnement de l'unité de contrôle (15) et, lors de la détection d'une anomalie de fonctionnement, amener l'unité de sortie (9) à sortir un état anormal,
l'unité de contrôle (15) est configurée pour effectuer un contrôle de fonctionnement de la première unité de commande (8) et, lors de la détection d'une anomalie de fonctionnement, amener l'unité de sortie (9) à sortir un état anormal,
la première unité de commande (8), l'unité de contrôle (15), et l'unité de sortie (9) sont des unités de traitement pouvant fonctionner par un système d'alimentation en énergie capable d'alimenter en énergie en continu,
la première unité de commande (8) peut fonctionner par une première batterie (16) capable d'alimenter en énergie en continu ou une batterie amovible (2) attachable et détachable,
l'unité de contrôle (15) peut fonctionner par une seconde batterie (17) capable d'alimenter en énergie en continu ou la batterie amovible (2),
l'appareil médical (1) comprend en outre une seconde unité de commande (7) pouvant fonctionner par la batterie amovible (2), la seconde unité de commande (7) commandant l'appareil médical (1), et
au moins une de la première unité de commande (8) et la seconde unité de commande (7) est configurée pour effectuer un contrôle de fonctionnement de l'autre unité de commande et, lors de la détection d'une anomalie de fonctionnement, amener l'unité de sortie (9) à sortir un état anormal.

2. Appareil médical selon la revendication 1, dans lequel l'unité de sortie (9) peut fonctionner par la première batterie (16), la seconde batterie (17), ou la batterie amovible (2).

3. Appareil médical (1) selon l'une quelconque des revendications 1 à 2,
dans lequel la première unité de commande (8) est configurée pour fonctionner par la batterie amovible (2) à la place de la première batterie (16) quand la batterie amovible (2) est disponible.

4. Appareil médical (1) selon l'une quelconque des revendications 1 à 3,
dans lequel au moins une de la première unité de commande (8) et de l'unité de contrôle (15) est configurée pour déterminer qu'une anomalie de fonctionnement s'est produite dans l'autre unité quand elle ne reçoit pas de signal de survie périodique en provenance de l'autre unité.

5. Appareil médical (1) selon la revendication 4,
dans lequel la première unité de commande (8) est configurée pour transmettre des informations de demande de réponse à la seconde unité de commande (7) et, quand elle ne reçoit pas d'informations de réponse appropriées correspondant aux informations de demande de réponse en provenance de la seconde unité de commande (7) à un moment prédéterminé, déterminer qu'une anomalie de fonctionnement s'est produite dans la seconde unité de commande (7).
